# EUROPEAN PATENT APPLICATION

(11) **EP 4 144 832 A1**
(43) Date of publication of application: **08.03.2023**
(21) Application number: 20926956.2
(22) Date of filing: 26.03.2020
(51) Int. Cl.: C12N 1/20, A23L 33/135, A61K 35/747, A61P 39/02, C12R 1/25, C12R 1/225

(54) **NOVEL LACTOBACILLUS PLANTARUM AND LACTIC ACID BACTERIA COMPOSITION AND USE THEREOF FOR TREATING OR PREVENTING HEAVY METAL RELATED DISEASES**

(71) Applicant: Native Biomedicals Ltd., Tainan City 744 (TW)
(72) Inventor: TSAI, Cheng-Chih, Tainan City, Taiwan 744 (TW)
(74) Representative: Lang, Christian
(86) International application number: PCT/CN2020/081259
(87) International publication number: WO 2021/189336

(57) **Abstract**

The invention provides a novel Lactobacillus plantarum NBM-01-07-003, which is scientifically named: Lactobacillus plantarum subsp. plantarum NBM-01-07-003, depositary authority: China General Microbiological Culture Collection Center (CGMCC) of the China Committee for Culture Collection of Microorganisms (CCCCM); address: Institute of Microbiology, Chinese Academy of Sciences, NO.1 Beichen West Road, Chaoyang District, Beijing 100101, China; depositary date: September 4, 2018; depositary number: CGMCC No. 16417. The novel Lactobacillus plantarum NBM-01-07-003 has a use of adsorbing metals, and is capable of achieving an efficacy of preventing or/and curing metal poisoning or related complications.

## Description

### BACKGROUND OF THE INVENTION

### Field of Invention

The invention relates to a second use of a microorganism, and more particularly to a novel Lactobacillus plantarum, a lactic acid bacteria composition and a use thereof for curing or preventing heavy metal related diseases.

### Related Art

Heavy metal pollution means that the environment is polluted by heavy metals or their compounds, which is mainly caused by man-made factors such as industrial emissions, exhaust gases discharged by vehicles, sewage, etc. Since heavy metals have the characteristics of not easy to move and dissolve, chronic poisoning can be caused when an individual is exposed to metal pollutants in the environment over a long period or in large-scale. At present, the most harmful heavy metals to human health include lead, mercury, arsenic, cadmium, and chromium.

The so-called lead poisoning refers to the poisoning phenomenon caused by human being exposed to lead or its compounds. Most of lead poisoning occurs through inhalation into the human body, such as motorcycle exhaust gases and air containing lead particles. Lead that enters into the human body is absorbed by the small intestine and stored in the body; when children come into contact with items containing lead, lead will be absorbed into the body through the panel. Because lead is not easy to be excreted from the body, long-term exposure will cause harm to various organs, especially have the greatest impact on the nervous system and digestive system. For example, long-term lead poisoning in children can cause brain damage, paralysis and other diseases.

It can be known from the above that heavy metals and their compounds are a major killer of human health, but exposure to heavy metals and their compounds in the environment cannot be avoided. Therefore, it is urgent to develop a product that is capable of effectively excreting heavy metals from the human body.

### SUMMARY OF THE INVENTION

A main object of the invention is to provide a novel Lactobacillus plantarum NBM-01-07-003, which is scientifically named: Lactobacillus plantarum subsp. plantarum NBM-01-07-003, depositary authority: China General Microbiological Culture Collection Center (CGMCC) of the China Committee for Culture Collection of Microorganisms (CCCCM); address: Institute of Microbiology, Chinese Academy of Sciences, NO.1 Beichen West Road, Chaoyang District, Beijing 100101, China; depositary date: September 4, 2018; depositary number: CGMCC No. 16417.

The Lactobacillus plantarum was also deposited at the Food Industry Research and Development Institute of the Hsinchu Foundation of Taiwan, China on December 15, 2017, with the depositary number: BCRC910809.

The novel Lactobacillus plantarum NBM-01-07-003 disclosed in the invention has a use of adsorbing metals, that is, the novel Lactobacillus plantarum NBM-01-07-003 is capable of adsorbing metals or their compounds in vivo or in vitro to achieve an efficacy of preventing or/and curing metal poisoning or related complications. Specifically, the novel Lactobacillus plantarum NBM-01-07-003 disclosed in the invention has a capacity to remove lead or its compounds.

In other words, by administering the novel Lactobacillus plantarum NBM-01-07-003 disclosed in the invention or a composition containing the novel Lactobacillus plantarum NBM-01-07-003 disclosed in the invention into a body of an individual, or by using the novel Lactobacillus plantarum NBM-01-07-003 disclosed in the invention or the composition containing the novel Lactobacillus plantarum NBM-01-07-003 disclosed in the invention in an environment, the novel Lactobacillus plantarum NBM-01-07-003 disclosed in the invention is capable of attaching on intestinal cells and panel cells, capable of effectively adsorbing heavy metal ions, and reducing the content of heavy metals in an organism or an environment in order to prevent or cure related diseases caused by heavy metals, such as central and peripheral nervous systems damage, hypomnesia and child intelligence decline, antisocial behavior, impaired hemoglobin synthesis, impaired renal function, deafness, blindness, hypoevolutism, loss of libido, inflammation, autoimmune diseases, etc. Therefore, the composition containing the novel Lactobacillus plantarum NBM-01-07-003 disclosed in the invention can be used as an effective ingredient of an external composition or an internal composition.

Wherein, the external composition can be a spray.

Wherein, the internal composition can be a food, a nutritional supplement, an additive.

In one embodiment disclosed in the invention, a lactic acid bacteria composition is provided, which comprises an effective amount of the novel Lactobacillus plantarum NBM-01-07-003 disclosed in the invention.

In another specific embodiment, the lactic acid bacteria composition further comprises a Lactobacillus delbrueckii subsp. lactis.

Specifically, the novel Lactobacillus plantarum NBM-01-07-003 disclosed in the invention and the Lactobacillus delbrueckii subsp. lactis can be mixed at a weight ratio of 1:1 to form a composition.

Further, the lactic acid bacteria composition disclosed in the above embodiments can have a use of adsorbing heavy metals, and can be prepared into a food, an external dosage form according to those skilled in the art to which the invention pertains, for applying to the human body or in vitro.

The lactic acid bacteria composition disclosed in the above embodiments has a use of curing or/and preventing heavy metal poisoning, that is, by administering an effective amount of the lactic acid bacteria composition to an individual, the lactic acid bacteria composition is capable of adsorbing heavy metals in the cells in order to achieve an efficacy of removing metals or their compounds absorbed into the human body, and thus capable of preventing or curing diseases caused by heavy metals.

### BRIEF DESCRIPTION OF THE DRAWINGS

FIG. 1 is the result of observing a novel Lactobacillus plantarum NBM-01-07-003 disclosed in the invention with an electron microscope;
FIG. 2 is the result of identification of the novel Lactobacillus plantarum NBM-01-07-003 disclosed in the invention with 16S rDNA;
FIG. 3 is the result of identification of a NBM-04-10-001 strain with 16S rDNA;
FIG. 4 is the result of attachment test of a lactic acid bacteria sample on human HaCaT cells, the upper figure is the result of attachment test of the NBM-04-10-001 strain, and the lower figure is the result of attachment test of a NBM-01-07-003 strain;
FIG. 5 is the result of attachment test of the lactic acid bacteria sample on human Caco-2 cells, the upper figure is the result of attachment test of the NBM-04-10-001 strain, and the lower figure is the result of attachment test of the NBM-01-07-003 strain;
FIG. 6 is the result of the lactic acid bacteria sample stimulating HaCaT cells to secrete IL-6;
FIG. 7 is the result of the lactic acid bacteria sample producing LPS to stimulate HaCaT cells to secrete IL-6;
FIG. 8 is the result of the lactic acid bacteria sample stimulating HaCaT cells to secrete IFN-Y;
FIG. 9 is the result of the lactic acid bacteria sample producing LPS to stimulate HaCaT cells to secrete IFN-Y;
FIG. 10 is the result of the lactic acid bacteria sample stimulating HaCaT cells to secrete IL-8;
FIG. 11 is the result of the lactic acid bacteria sample producing LPS to stimulate HaCaT cells to secrete IL-8;
FIG. 12 is the result of the lactic acid bacteria sample stimulating Caco-2 cells to secrete IL-8;
FIG. 13 is the result of the lactic acid bacteria sample producing LPS to stimulate Caco-2 cells to secrete IL-8;
FIG. 14 is the result of the lactic acid bacteria sample stimulating mouse macrophages RAW264.7 to secrete IL-6;
FIG. 15 is the result of the lactic acid bacteria sample producing LPS to stimulate mouse macrophages RAW264.7 to secrete IL-6;
FIG. 16 is the result of the lactic acid bacteria sample stimulating mouse macrophages RAW264.7 to secrete TNF-α;
FIG. 17 is the result of the lactic acid bacteria sample producing LPS to stimulate mouse macrophages RAW264.7 to secrete TNF-α;
FIG. 18 is the result of observing bacteria that have not undergone heavy metal test with an electron microscope;
FIG. 19 is the result of observing bacteria that have undergone heavy metal test with an electron microscope;
FIG. 20 is the result of staining liver sections of rats in first to third groups after different treatments;
FIG. 21A is the result of staining the liver sections of the rats in the first group;
FIG. 21B is the result of staining the liver sections of the rats in the second group; and
FIG. 21C is the result of staining the liver sections of the rats in the third group.

### DETAILED DESCRIPTION OF THE INVENTION

A novel Lactobacillus plantarum NBM-01-07-003 (hereinafter referred to as NBM-01-07-003) disclosed in the invention is scientifically named: Lactobacillus plantarum subsp. plantarum NBM-01-07-003, depositary authority: China General Microbiological Culture Collection Center (CGMCC) of the China Committee for Culture Collection of Microorganisms (CCCCM); address: Institute of Microbiology, Chinese Academy of Sciences, NO.1 Beichen West Road, Chaoyang District, Beijing 100101, China; depositary date: September 4, 2018; depositary number: CGMCC No. 16417. The Lactobacillus plantarum was also deposited at the Food Industry Research and Development Institute of the Hsinchu Foundation of Taiwan, China on December 15, 2017, with the depositary number: BCRC910809.

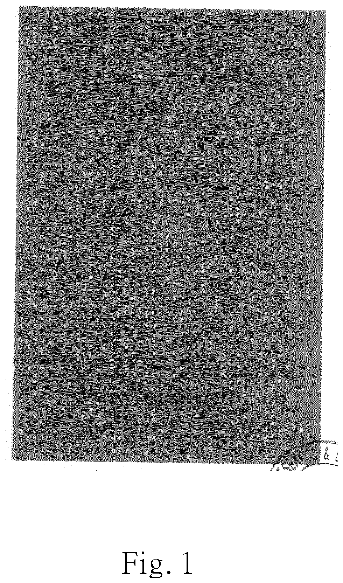

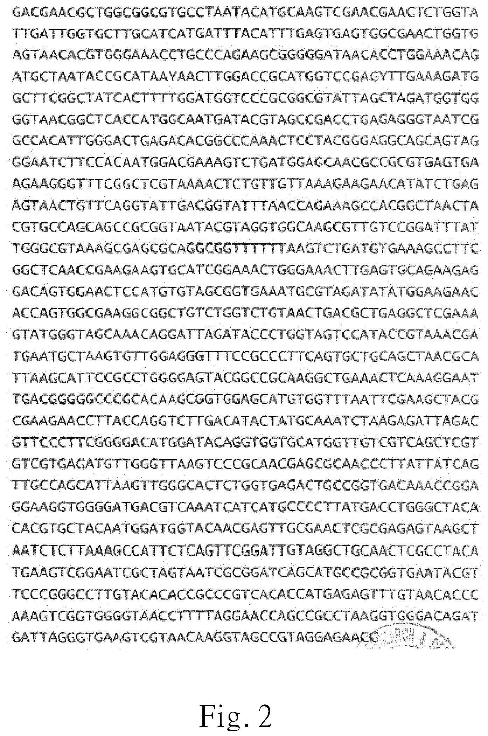

Further, please refer to FIG. 1. The novel Lactobacillus plantarum NBM-01-07-003 disclosed in the invention is isolated from infant feces, the NBM-01-07-003 is a Gram-positive bacillus, does not have catalase, oxidase, and motility, does not produce endospores, and will grow in aerobic or anaerobic environment. The novel Lactobacillus plantarum NBM-01-07-003 can be cultured in MRS medium at 37°C. The result of identification of the novel Lactobacillus plantarum NBM-01-07-003 with 16S rDNA is shown in FIG. 2, that is Seq ID No. 1. Comparison shows that the novel Lactobacillus plantarum NBM-01-07-003 disclosed in the invention is close to Lactobacillus plantarum subsp. plantarum, Lactobacillus plantarum subsp. argentoratensis, Lactobacillus plantarum and Lactobacillus plantarum subsp. fabifermentant, with a similarity of 99%, that is, the novel Lactobacillus plantarum NBM-01-07-003 disclosed in the invention is a novel strain.

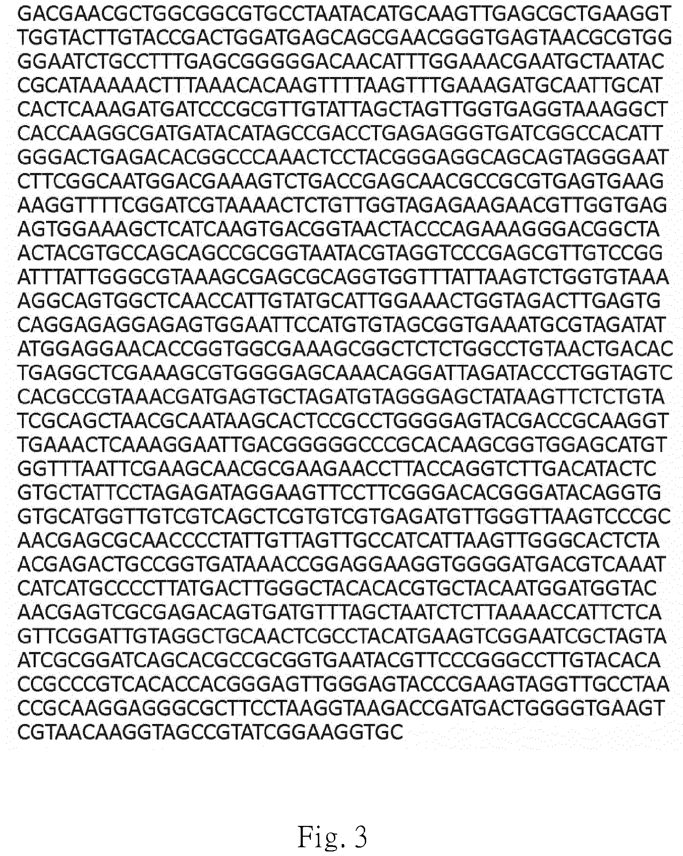

The Lactobacillus delbrueckii subsp. lactis used in an example of the invention is named NBM-04-10-001 (hereinafter referred to as NBM-04-10-001), which is isolated from fermented milk and is a Gram-positive bacillus, does not have catalase, oxidase, and motility, does not produce endospores, and will grow in aerobic or anaerobic environment. Please refer to FIG. 3, which is Seq ID No. 2. After 16S rDNA sequence analysis and API identification system analysis, it can be known that the NBM-04-10-001 strain used in the example of the invention is the Lactobacillus delbrueckii subsp. lactis, which is a commercially available microorganism (the depositary number is ATCC 12315, purchased from the Food Industry Research and Development Institute of Taiwan), so deposit is not required.

In the following, several examples in conjunction with tables will be used to further illustrate the efficacies of the invention.

### Example 1: Attachment test of lactic acid bacteria

Take a 24-well culture plate, add 1ml of cell suspension to each well, adjust the cell concentration to 5×104cell/mL, mix evenly and place it in a 37°C, 5% carbon dioxide incubator to culture overnight to allow the cells to attach and grow. After confirming that the cells are completely attached, aspirate the old medium, wash with phosphate buffer solution, add 900µL of fresh medium and 100µl of a lactic acid bacteria sample (107CFU/mL) re-dissolved in the cell culture medium, and then place it in a 37°C, 5% carbon dioxide incubator to culture for 2 hours, aspirate the culture solution and wash with phosphate buffer solution, and then add 200µL of 6-10% formalin to each well to react for 30 minutes to fix the bacteria and the cells in the wells. Remove the formalin, wash with phosphate buffer solution, add 200µL of crystal violet to react for 5 minutes to stain, and observe and count a quantity of lactic acid bacteria on the cells with an inverted fluorescent microscope.

In this example, the cells used are human rectal cancer cell lines C2BBel (hereinafter referred to as human cell lines Caco-2) and human immortalized panel keratinocyte cell lines HaCaT (hereinafter referred to as human cell lines HaCaT); the medium is DMEM (Dulbecco's Modified Eagle Medium) medium; the lactic acid bacteria are NBM-04-10-001, NBM-01-07-003.

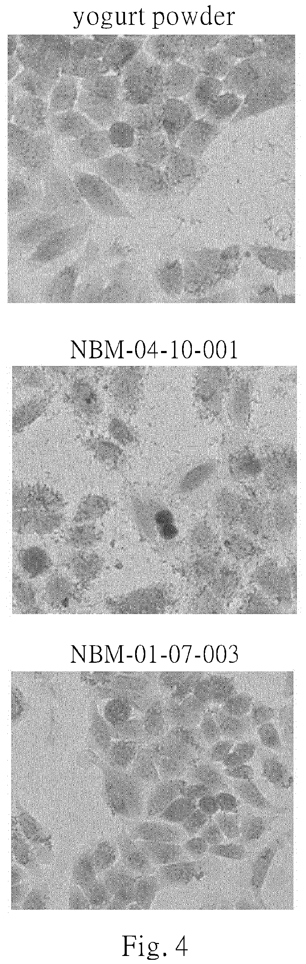

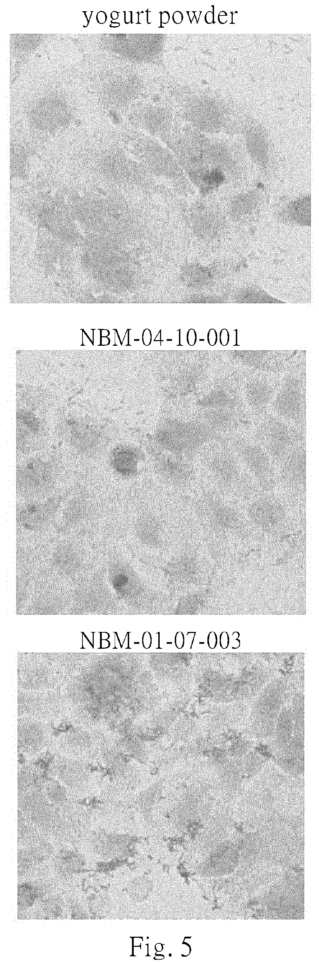

The results are shown in Table 1 and Table 2 as well as FIGS. 4 and 5.

**Table 1: Attachment capacity of lactic acid bacteria on human cell lines HaCaT**

| Lactic acid bacteria sample | Quantity of attachment |
|---|---|
| NBM-04-10-001 | Too many, uncountable |
| NBM-01-07-003 | 36.2±7.66 |

**Table 2: Attachment capacity of lactic acid bacteria on human cell lines Caco-2**

| Lactic acid bacteria sample | Quantity of attachment |
|---|---|
| NBM-04-10-001 | 42.1±9.72 |
| NBM-01-07-003 | 50.6±5.58 |

Since the previous documents pointed out that when more than 15e lactic acid bacteria are attached to the squamous epithelial cells of pigs, it can be considered that the lactic acid bacteria have a capability to attach. Therefore, from the results of Tables 1 and 2 and FIGS. 4 and 5, it can be known that the lactic acid bacteria samples used in this example have good adhesion on human cell lines HaCaT and human cell lines Caco-2 respectively. Wherein, NBM-04-10-001 has better adhesion on the human cell lines HaCaT; NBM-01-07-003 has better adhesion on the human cell lines Caco-2.

Therefore, it can be known from the above results that the novel Lactobacillus plantarum NBM-01-07-003 disclosed in the invention has good adhesion on the intestinal tract, which shows that the novel Lactobacillus plantarum NBM-01-07-003 disclosed in the invention is capable of exerting efficacies in vivo or in vitro.

### Example 2: Immunity test of lactic acid bacteria on cells

Cultivate the cells in a 24-well culture plate, add a predetermined concentration of 1ml cell suspension to each well, and then add 100µL of lactic acid bacteria solution, quantities of bacteria are 109, 108, 107, 106, 105cfu/mL respectively, or add LTA lysis solution for co-cultivation for 24 hours, the supernatant is collected at the end of the acting time and analyzed with a commercially available ELISA kit.

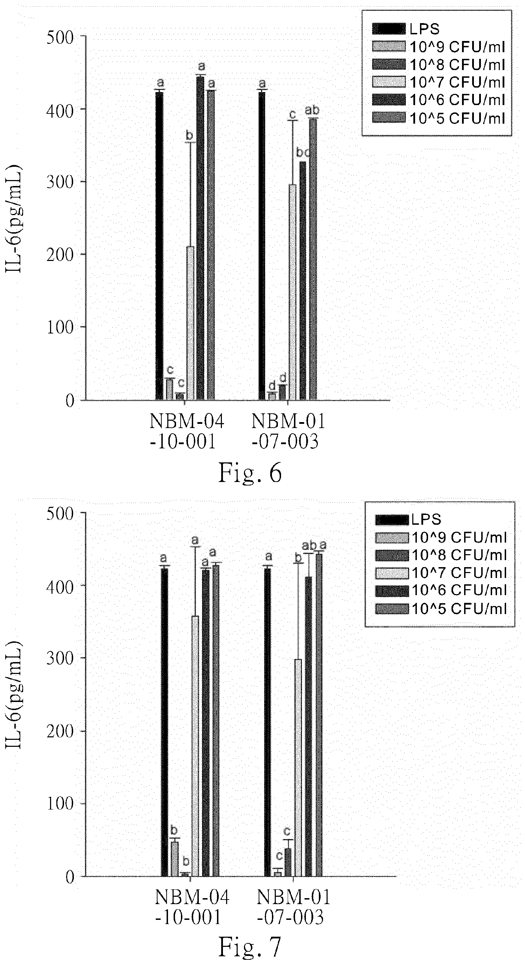

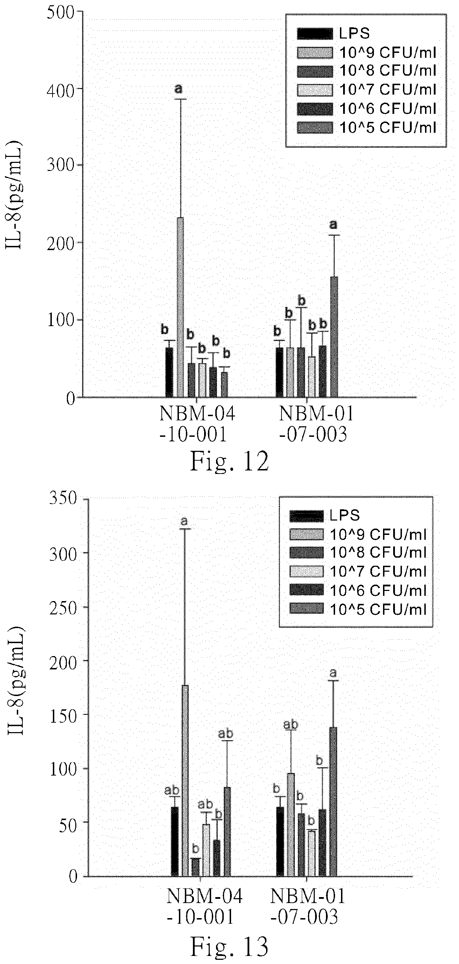

In this example, the cells used for the test and their concentrations are respectively: human CaCo-2 cells, concentration is adjusted to 1 × 105 cell/ml; human HaCaT cells, concentration is adjusted to 9×104 cell/ml; and mouse macrophages RAW264.7, concentration is adjusted to 2×105cell/ml. In addition, the lactic acid bacteria solution used is obtained from NBM-04-10-001 and NBM-01-07-003, and the results are shown in FIGS. 6-17.

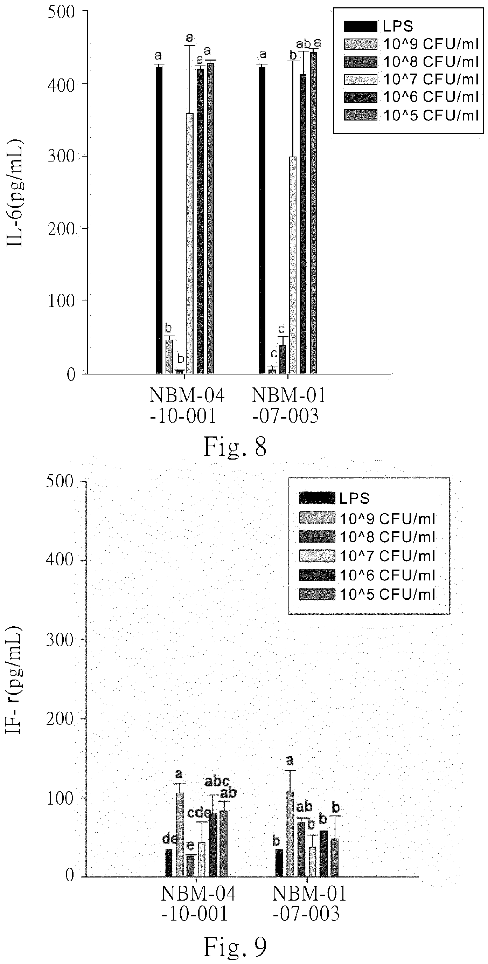

From the results in FIG. 6 and FIG. 7, it can be known that whether human HaCaT cells directly react with the lactic acid bacteria or react after being stimulated by the lactic acid bacteria producing LPS, NBM-04-10-001 and NBM-01-07-003 are capable of effectively reducing a secretory volume of IL-6 when a quantity of bacteria is 109 or 108 cfu/ml, showing that the NBM-01-07-003 disclosed in the invention is capable of reducing stimulation of the cells to produce immunoreaction; from the results in FIG. 8 and FIG. 9, it can be known that after the NBM-04-10-001 of 109 cfu/ml is co-cultured with human HaCaT cells, a secretory volume of the cytokine IFN-Y can be significantly increased, thereby achieving an efficacy of reducing the expression of IL-4 and Th1-mediated inflammatory response.

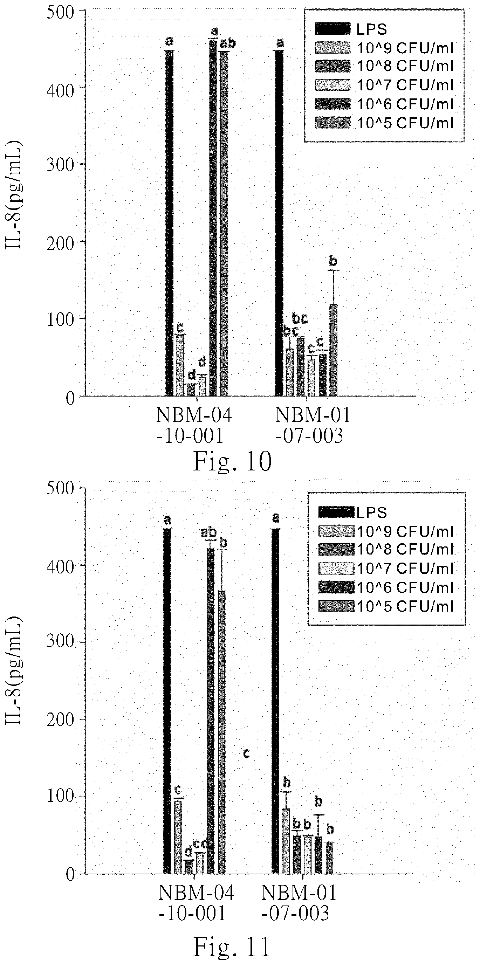

From the results in FIG. 10 and FIG. 11, it can be known that LAB-001 of 109, 108, 107 cfu/ml or the NBM-04-10-001 of 109, 108, 107, 106, 105 cfu/ml and the human cell lines HaCaT are capable of significantly reducing a secretory volume of IL-8 by the cells and reducing the occurrence of inflammatory response and angiogenesis. From the results in FIG. 9 and FIG. 10, it can be known that NBM-04-10-001 and NBM-01-07-003 are capable of effectively reducing a secretory volume of IL-8 when a quantity of bacteria is 108, 107, 106 cfu/ml.

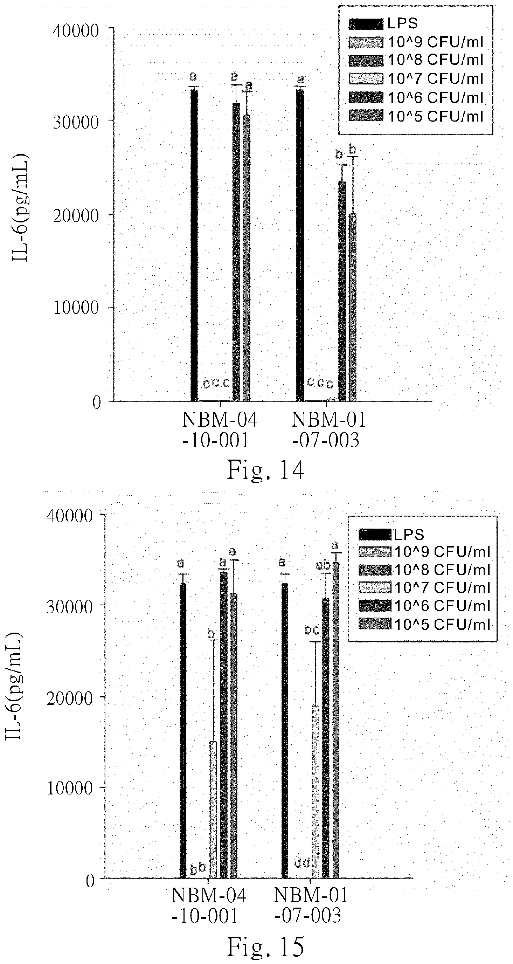

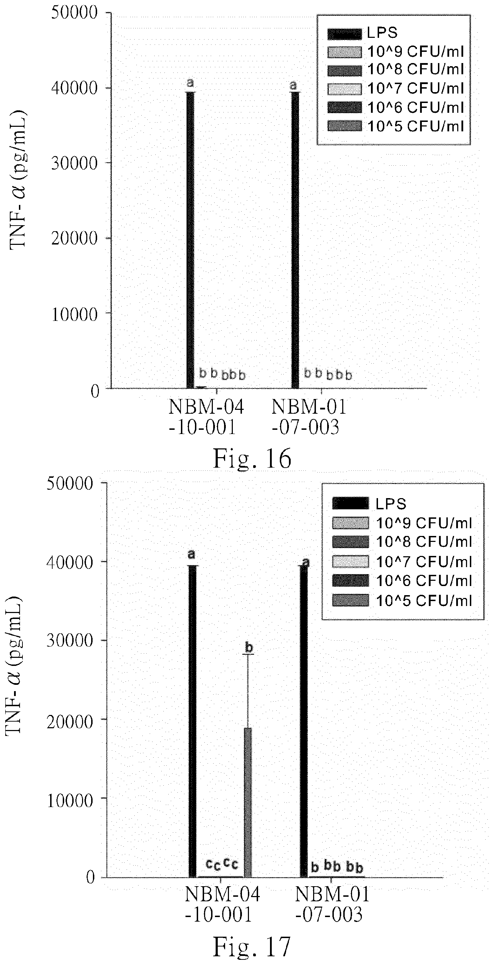

Further, it can be known from the results in FIG. 14 to FIG. 15 that for the mouse macrophages RAW264.7 which secrete immune factors, NBM-04-10-001 and NBM-01-07-003 are capable of reducing a secretory volume of IL-6, and as a quantity of bacteria increases, the better the reduction effect. From the results in FIG. 16 and FIG. 17, it can be known that regardless of whether the mouse macrophages RAW264.7 are stimulated by LPS, the added NBM-04-10-001 and NBM-01-07-003 are capable of reducing TNF-α.

### Example 3: Lead adsorption test

This example uses lead adsorption solution to culture with water, EDTA, NBM-04-10-001 and NBM-01-07-003, commercially available yogurt powder, and samples to be tested respectively. After cultivation, an absorbance is measured with a spectrophotometer, and the absorbance is converted to obtain a lead removal rate, and the results are shown in Table 3 below.

**Table 3: Lead removal rate**

| | Removal rate(%) | Removal rate(%) | Removal rate(%) | Average Removal rate (%) | SD value |
|---|---|---|---|---|---|
| Water | 0.0 | 0.0 | 0.0 | 0.0 | 0.0 |
| EDTA | 105.4 | 110.6 | 103.2 | 106.4 | 3.8 |
| NBM-04-10-001 | 75.5 | 86.2 | 80.0 | 80.6 | 5.4 |
| NBM-01-07-003 | 88.1 | 91.0 | 91.1 | 90.0 | 1.7 |

It can be known from the results in Table 3 that the lactic acid bacteria of NBM-04-10-001 and NBM-01-07-003 disclosed in the invention indeed are capable of effectively adsorbing lead and achieving an efficacy of removing lead.

### Example 4: In vitro test

Take the NBM-04-10-001 strain and the NBM-01-07-003 strain, and mix the two strains at a weight ratio of 1:1 to form a mixed bacterial powder.

Take 0.1g of the mixed bacterial powder and re-dissolve it in 1mL of deionized water, add 1mL to 10mL of 1ppm lead standard solution, and react at 37°C for 1 hour. Centrifuge the heavy metal suspension at 12000 rpm for 5 minutes to remove the precipitate (bacterial sludge) and take out the supernatant. The supernatant is filtered with a 0.22µm filtration membrane and analyzed with an inductively coupled plasma atomic emission spectrometer (ICP-OES), and the result shows that a capability of the mixed bacterial powder in removing 1ppm lead metal ions in 10mL: 57.21±0.06%. It can be known that the NBM-01-07-003 strain disclosed in the invention or the composition containing the NBM-01-07-003 strain disclosed in the invention indeed is capable of adsorbing more than 50% of lead metal ions in a 1 ppm lead solution.

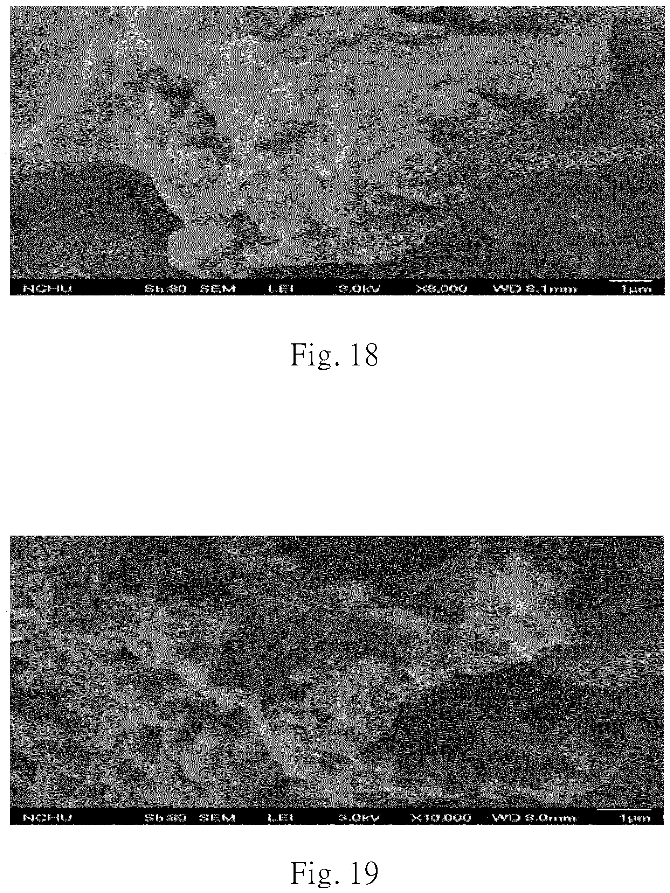

In addition, the bacterial sludge is placed in a centrifuge test tube for freeze-drying. The observed bacterial flora of the dried bacterial sludge are divided into control group (have not undergone heavy metal adsorption test) and heavy metal test group (have undergone heavy metal adsorption test) in order to observe changes in adsorption of heavy metal lead by the bacteria. The result shows that the bacterial powder that adsorbs heavy metals is closely connected (as shown in FIG. 18), and in the bacterial powder that adsorbs heavy metals, although the bacteria are intact, surfaces of the bacteria are attached with broken substances and produce viscous substances, showing that the NBM-01-07-003 strain disclosed in the invention indeed is capable of adsorbing and aggregating heavy metals (as shown in FIG. 19).

### Example 5: Animal test (1)

Take six-week-old Wistar male rats (purchased from BioLASCO Taiwan Co., Ltd.) and divide them into 3 groups with 6 rats in each of the groups, wherein, the first group is the blank group; the second group is the lead nitrate poisoning group, a lead nitrate dose is 1/50 rat median lethal dose (LD50): 45mg/kg; and the third group is the mixed bacterial powder group, an administering dose of lead nitrate is 45mg/kg, and a mixed bacterial powder dose is 20-21mg/kg/day, wherein the mixed bacterial powder contains the NBM-04-10-001 strain and the NBM-01-07-003 strain, and the two strains are mixed at a weight ratio of 1:1, and the mixed bacterial powder is fed orally two hours after the administration of lead nitrate.

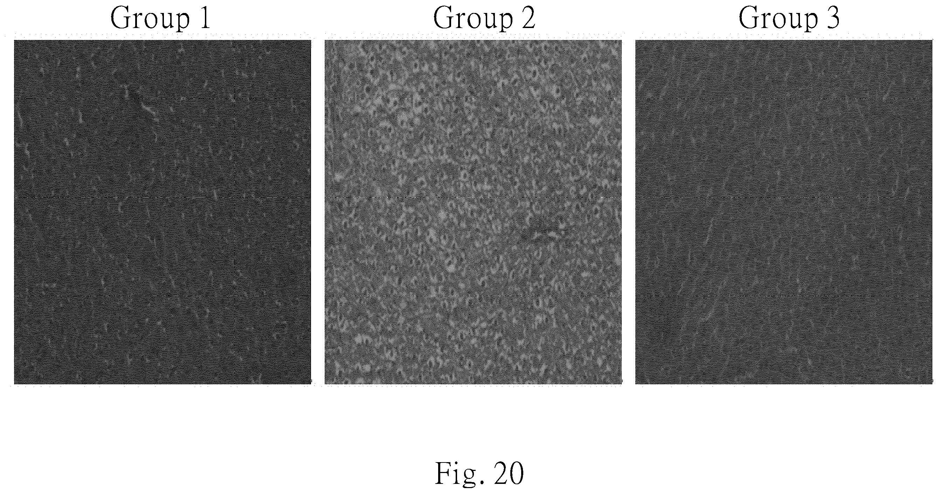

During the experiment, weight changes of the rats in each of the groups are measured, and the results are shown in Table 4. In addition, blood is collected every two weeks to detect lead content in the blood of the rats in each of the groups, and the results are shown in Table 5, wherein ND represents lead content in the blood is less than 0.1ppb. After the experiment, the rats in each of the groups are sacrificed, their livers and kidneys are sectioned and observed, and the results are shown in FIG. 20.

It can be known from the results in Table 4 below that there is no significant difference in the ingestion volume of the rats in the first to third groups.

From the results in Table 5, it can be known that lead content in the blood of the rats in the second group is 2.5~3.1ppb, and compared with the rats in the second group, no lead is detected in the blood of the rats in the third group, showing that administration of the NBM-01-07-003 strain disclosed in the invention or the composition containing the NBM-01-07-003 strain disclosed in the invention is capable of effectively removing and adsorbing the heavy metal lead content in animal blood.

From the results in FIG. 20, it can be known that the hepatocytes of the first group of rats are closely arranged, and the nucleoli are obvious, no cell damage, swelling, nuclear lysis symptoms, and vacuoles; pores in the hepatocytes of the rats in the second group become larger distinctly, and there are small local areas of necrotic cells, karyopycnosis and lysis, indicating that the hepatocytes of the second group of rats are affected by lead and resulting in liver injury; compared with the second group of rats, damage in the hepatocytes of the rats administered with the composition containing the NBM-01-07-003 strain disclosed in the invention is significantly reduced, even similar to the hepatocytes of the first group of rats, indicating that the NBM-01-07-003 strain disclosed in the invention or the composition containing the NBM-01-07-003 strain disclosed in the invention is not only capable of removing lead in the tissues, preventing lead from accumulating in the tissues or cells, but also capable of reducing oxidation reaction of the tissues or cells, and reversing damage or pathological changes caused by heavy metals to organs.

**Table 4: Changes in body weight and ingestion volume of rats in each group**

| Group | Weight (g) | | | | |
|---|---|---|---|---|---|
| | Start | Final | Weight gain percentage (%) | Daily weight gain (g) | Food intake (g/day) |
| 1 | 194±58.67 | 353.82±34.82 | 158.82% | 11.42±2.37 | 57.6±12.59 |
| 2 | 240.17±17.28 | 374.33±23.04 | 134.16% | 9.58±0.97 | 58.12±11.43 |
| 3 | 240.33±9.63 | 355.63±16.84 | 147.97% | 8.24±0.75 | 54.54±12.10 |

**Table 5: Lead content in blood of rats in each group**

| Group | Lead content in blood (ppb) | | | | | |
|---|---|---|---|---|---|---|
| | 1st rat | 2nd rat | 3rd rat | 4th rat | 5th rat | 6th rat |
| 1 | ND | ND | ND | ND | ND | ND |
| 2 | 3.1 | 2.9 | 2.5 | 2.1 | 2.5 | 2.6 |
| 3 | ND | ND | ND | ND | ND | ND |

### Example 6: Animal experiment (2)

The design of this example is roughly the same as that of example 5, but the difference is that lead nitrate is replaced with arsenic nitrate, and an administering dose is 1/40 rat median lethal dose (LD50) : 1 mg/kg. To put it simply, in this example, the rats are divided into 3 groups, wherein, the first group is the blank group; the second group is the arsenic nitrate poisoning group, a dose of arsenic nitrate: 1mg/kg; and the third group is the mixed bacterial powder group, an administering dose of arsenic nitrate is 1 mg/kg, and a dose of the mixed bacterial powder is 20-21mg/kg/day, wherein, the mixed bacterial powder contains the NBM-04-10-001 strain and the NBM-01-07-003 strain, and the two strains are mixed at a weight ratio of 1:1, and the mixed bacterial powder is fed orally two hours after the administration of arsenic nitrate. The test duration of this example is 3 weeks.

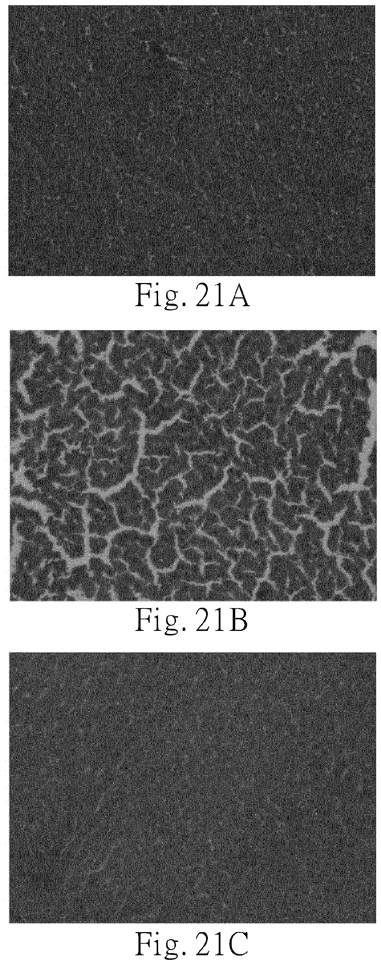

In the third week of the test, blood is collected from the eye sockets of the rats in each of the groups to detect arsenic content in the blood, and the results are shown in Table 6 below. After the test, the rats in each of the groups are sacrificed, and their livers are taken, sectioned and stained, and the results are shown in FIG. 21.

From the results in Table 6 and FIG. 21, it can be known that the composition containing the NBM-01-07-003 strain disclosed in the invention is capable of effectively removing arsenic in the blood, and preventing arsenic from accumulating in the liver, so that the hepatocytes will not be damaged by arsenic. As shown in FIG. 21, administration of the composition containing the NBM-01-07-003 strain disclosed in the invention is capable of arranging the hepatocytes closely without necrocytosis, karyopycnosis and lysis.

This shows that the NBM-01-07-003 strain disclosed in the invention or the composition containing the NBM-01-07-003 strain disclosed in the invention is capable of removing arsenic from the blood and preventing arsenic from accumulating in the liver or other tissues in order to achieve an efficacy of preventing organs from being damaged by heavy metals.

**Table 6: Changes of arsenic content in blood of rats in each group**

| Group | Arsenic content in blood (ppb) | | | | | |
|---|---|---|---|---|---|---|
| | 1st rat | 2nd rat | 3rd rat | 4th rat | 5th rat | 6th rat |
| 1 | ND | ND | ND | ND | ND | ND |
| 2 | 2.5 | 2.3 | 2.9 | 2.5 | 2.8 | 2.6 |
| 3 | ND | ND | ND | ND | ND | ND |

According to the above description, it can be confirmed that the novel Lactobacillus plantarum NBM-01-07-003 disclosed in the invention indeed is capable of attaching on intestinal cells or panel cells, not only capable of reducing individual inflammation, regulating immunoreaction, but also can be different from conventional lactic acid bacteria, capable of adsorbing heavy metals such as lead and arsenic or compositions containing heavy metals in vivo or in vitro, and can be used as an active ingredient in a composition for curing or/and preventing related diseases or complications caused by heavy metals. Further, the novel Lactobacillus plantarum NBM-01-07-003 disclosed in the invention is capable of cooperating with other lactic acid bacteria, such as NBM-04-10-001 in order to further enhance its capability of adsorbing heavy metals and achieve a better removing efficacy, for example, the novel Lactobacillus plantarum NBM-01-07-003 and NBM-04-10-001 disclosed in the invention can be mixed according to a predetermined ratio, such as 1:1, 2:1, 3:1, 1:2, 1:3, etc.

It is to be understood that the above description is only the embodiments and examples of the invention and is not used to limit the present invention, and changes in accordance with the concepts of the present invention may be made without departing from the spirit of the present invention. For example, the equivalent effects produced by various transformations, variations, modifications and applications made to the configurations or arrangements shall still fall within the scope covered by the appended claims of the present invention.

## Claims

1. A novel Lactobacillus plantarum NBM-01-07-003, **characterized in that**, scientifically named: Lactobacillus plantarum subsp. plantarum NBM-01-07-003, depositary authority: China General Microbiological Culture Collection Center (CGMCC) of the China Committee for Culture Collection of Microorganisms (CCCCM); address: Institute of Microbiology, Chinese Academy of Sciences, NO.1 Beichen West Road, Chaoyang District, Beijing 100101, China; depositary date: September 4, 2018; depositary number: CGMCC No. 16417.

2. A lactic acid bacteria composition, **characterized in that**, comprising the novel Lactobacillus plantarum NBM-01-07-003 as claimed in claim 1.

3. The lactic acid bacteria composition as claimed in claim 2, **characterized in that**, further comprising a Lactobacillus delbrueckii subsp. lactis.

4. The lactic acid bacteria composition as claimed in claim 3, **characterized in that**, the novel Lactobacillus plantarum NBM-01-07-003 and the Lactobacillus delbrueckii subsp. lactis are mixed at a weight ratio of 1:1.

5. The lactic acid bacteria composition as claimed in claim 2 or 3, **characterized in that**, being used for adsorbing heavy metals.

6. The lactic acid bacteria composition as claimed in claim 2 or 3, **characterized in that**, being prepared into an external dosage form.

7. A use of a lactic acid bacteria composition for manufacturing a composition for curing or/and preventing heavy metal poisoning, **characterized in that**, the lactic acid bacteria composition comprises the novel Lactobacillus plantarum NBM-0 1-07-003 as claimed in claim 1.

8. The use as claimed in claim 7, **characterized in that**, the lactic acid bacteria composition further comprises a Lactobacillus delbrueckii subsp. lactis.

9. The use as claimed in claim 8, **characterized in that**, the novel Lactobacillus plantarum NBM-01-07-003 and the Lactobacillus delbrueckii subsp. lactis are mixed at a weight ratio of 1:1.

10. The use as claimed in claim 7, 8 or 9, **characterized in that**, the lactic acid bacteria composition is a food.
